# EUROPEAN PATENT APPLICATION

(11) **EP 3 348 196 A1**
(43) Date of publication of application: **18.07.2018**
(21) Application number: 16844598.9
(22) Date of filing: 18.08.2016
(51) Int. Cl.: A61B 6/00, G01T 7/00

(54) **X-RAY DETECTOR, X-RAY IMAGING DEVICE, AND CONTROL METHOD THEREFOR**

(30) Priority: 08.09.2015 KR 20150126677
(71) Applicant: Samsung Electronics Co., Ltd., Suwon-si, Gyeonggi-do 16677 (KR)
(72) Inventor: KIM, Myeong Je, Seoul 05820 (KR)
(74) Representative: Gulde & Partner
(86) International application number: PCT/KR2016/009100
(87) International publication number: WO 2017/043778

(57) **Abstract**

Disclosed herein is an X-ray detector capable of detecting its storage position, an X-ray imaging apparatus having the same and a control method for the same. The X-ray imaging apparatus determines which X-ray detector is stored in which detector storage position and precisely provides charging information of the X-ray detector to a user.

The X-ray detector comprises a detecting portion configured to collect storage position information of the X-ray detector and a communicator configured to transmit the storage position information to the X-ray imaging apparatus.

## Description

### [Technical Field]

The present disclosure relates to an X-ray detector capable of detecting a storage position, an X-ray imaging apparatus having the same and a control method for the same

### [Background Art]

X-ray imaging apparatuses are apparatuses that obtain an image inside an object using X-rays. The X-ray imaging apparatuses capture an image inside the object using non-invasive methods, whereby X-rays are radiated onto the object and the X-rays transmitted by the object are detected. Thus, these X-ray imaging apparatuses for medical purposes can be used to diagnose an injury or a disease inside the object that cannot be checked from the exterior.

The X-ray imaging apparatuses include an X-ray source that generates X-rays and radiates the X-rays onto the object, and an X-ray detector that detects X-rays transmitted through the object. The X-ray source may be movably disposed so as to image various parts of the object. The X-ray detector can be mounted on an image capturing table or an image capturing stand, or the X-ray detector can be used in a portable manner

The X-ray imaging apparatus may be provided with a plurality of detector storage portions to store and to charge the X-ray detector. When the X-ray detector is charged in a plurality of storage portions, it may be required to precisely distinguish which X-ray detector is mounted to which detector storage portion in order to display a state of charge of the detector storage portion.

### [Disclosure]

### [Technical Problem]

The present disclosure is directed to providing an X-ray detector capable of detecting a storage position, an X-ray imaging apparatus having the same and a control method for the same.

### [Technical Solution]

In accordance of the present disclosure there is provided an X-ray detector. The X-ray detector includes a detecting portion configured to collect storage position information of the X-ray detector; and a communicator configured to transmit the storage position information to the X-ray imaging apparatus.

The detecting portion may include at least one of an acceleration sensor or a magnetic sensor

The acceleration sensor may collect information about a gradient of the X-ray detector.

The magnetic sensor may collect information about magnetic flux around the X-ray detector.

The detector storage portion may comprise a first detector storage portion and a second detector storage portion adjacent to the first detector storage portion.

The first detector storage portion may be provided to be inclined at a predetermined first angle with the second detector storage portion.

A magnet may be provided between the first detector storage portion and the second detector storage portion.

The X-ray detector may further comprise a memory configured to store identification (ID) information of the X-ray detector.

One aspect of the present disclosure there is provided an X-ray imaging apparatus. The X-ray imaging apparatus include a plurality of detector storage portions configured to store an X-ray detector; and a controller when the X-ray detector is stored in the detector storage portion, configured to determine the corresponding X-ray detector is stored in which detector storage portion between the plurality of detector storage portions, based on position information of the X-ray detector, which is transmitted from the X-ray detector.

The X-ray detector may comprise a detecting portion configured to collect position information of the X-ray detector and a communicator configured to transmit the position information to the X-ray imaging apparatus.

The detecting portion may include at least one of an acceleration sensor or a magnetic sensor

The acceleration sensor may collect information about a gradient of the X-ray detector.

The controller may determine a storage position of the X-ray detector based on the gradient information.

The magnetic sensor may collect information about magnetic flux around the X-ray detector

The controller may determine a storage position of the X-ray detector based on the magnetic flux information.

The plurality of detector storage portion may comprise a first detector storage portion and a second detector storage portion adjacent to the first detector storage portion.

The first detector storage portion may be provided to be inclined at a predetermined first angle with the second detector storage portion.

A magnet may be provided between the first detector storage portion and the second detector storage portion.

The controller matches ID information of the X-ray detector, which is pre-stored, with ID information of the X-ray detector, which is transmitted from the X-ray detector, and determines a storage position of the X-ray detector.

The X-ray imaging apparatus may further comprise display configured to display charging information of the X-ray detector stored in the detector storage portion.

The display may display the charging information of the X-ray detector stored in the plurality of storage portions.

The display may include a display of a workstation.

Another aspect of the present disclosure provides a control method of an X-ray imaging apparatus having a plurality of detector storage portions in which an X-ray detector is stored, including receiving ID information and storage position information of the X-ray detector, from the X-ray detector; and determining the X-ray detector is stored in which detector storage portion between the plurality of detector storage portions, based on the ID information and the storage position information of the X-ray detector.

The control method may further include displaying charging information of the X-ray detector stored in the detector storage portion

### [Advantageous Effects]

It is possible to determine which X-ray detector is stored in which detector storage portion and thus a user can be provided with charging information of the X-ray detector.

### [Description of Drawings]

FIG. 1 is an exterior view illustrating a mobile X-ray imaging apparatus 100 in accordance with an embodiment.
FIG. 2 is a perspective view illustrating the operator 125 of the X-ray imaging apparatus 100.
FIGS. 3 and 4 are views illustrating examples of the detector storage portion 150 of the X-ray imaging apparatus 100.
FIG. 5 is a control block diagram illustrating the X-ray imaging apparatus 100 in accordance with an embodiment.
FIG. 6 is a view illustrating an example of a screen (S) of the display displaying charge information of the X-ray detector 130.
FIG. 7 is a view illustrating a detailed configuration of the controller 176 according to FIG 5.
FIG. 8 is a view illustrating an example of a detector storage portion of the X-ray imaging apparatus according to another embodiment.
FIG. 9 is a control block diagram of the X-ray imaging apparatus of FIG. 8.
FIG. 10 is a view illustrating an example of a detector storage portion of the X-ray imaging apparatus according to another embodiment.
FIG. 11 is a control block diagram of the X-ray imaging apparatus of FIG. 10.
FIG. 12 is a view schematically illustrating an appearance of an X-ray imaging apparatus in the ceiling type.
FIG. 13 is a view illustrating an image capturing table of the X-ray imaging apparatus of FIG. 12.
FIG. 14 is a view illustrating an image capturing stand of the X-ray imaging apparatus of FIG. 12.
FIG. 15 is a view illustrating a connection structure between the X-ray detector and the workstation in accordance with an embodiment.
FIG. 16 is a view illustrating a signal flow of the X-ray detector and the workstation in accordance with an embodiment.
FIG. 17 is a view illustrating a signal flow of the X-ray detector and the workstation in accordance with another embodiment.

### [Mode for Invention]

Embodiments described in the present disclosure and configurations shown in the drawings are merely examples of the embodiments of the present disclosure, and may be modified in various different ways at the time of filing of the present application to replace the embodiments and drawings of the present disclosure.

Hereinafter, exemplary embodiments of an X-ray detector, an X-ray imaging apparatus having the same and a control method for the same will be described in detail with reference to the accompanying drawings. The same reference numerals or signs shown in the drawings of the present disclosure indicate elements or components performing substantially the same function.

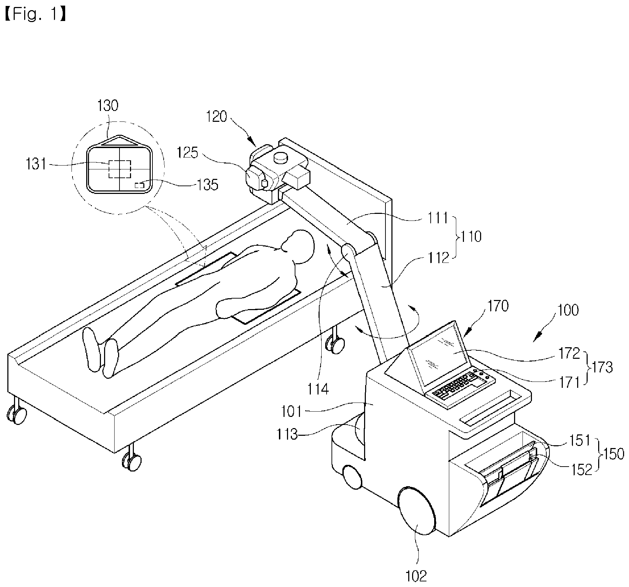

FIG. 1 is an exterior view illustrating a mobile X-ray imaging apparatus 100 in accordance with an embodiment.

Referring to FIG. 1, a mobile X-ray imaging apparatus 100 may include a body 101, a support arm 110, an X-ray source 120, an operator 125, an X-ray detector 130, a detector storage portion 150, and a workstation 170.

The body 101 may be provided movably. A wheel 102 configured to move the body 101 may be mounted on a lower end portion of the body 101.

The support arm 110 is configured to move the x-ray source 120 toward an object. The support arm 110 may be mounted on the movable body 101, and since a source connecting portion is formed on the end portion of the support arm 110, the x-ray source 120 may be mounted to the end portion of the support arm 110.

The support arms 110 may be provided to be rotatable in a direction parallel to the ground with respect to a mounting portion 113. The support arm 110 includes a first support arm 111 to which the x-ray source 120 is mounted and a second support arm 112 mounted to the body 101. The first support arm 111 and the second support arm 112 may meet at an arm connecting portion 114 and an inclination angle of the first support arm 111 and the second support arm 112 may be adjusted with respect to the arm connecting portion 114 and the mounting portion 113, respectively. Thus, the x-ray source 120 may move freely in any three-dimensional space.

The support arm 110 is not limited to that shown in FIG. 1. The first support arm 111 and the second support arm 112 may be integrally formed or alternatively, an auxiliary support arm may be further added as well as the first support arm 111 and the second support arm 112.

The X-ray source 120 is an apparatus that radiates X-rays onto an object. The X-ray source 120 may include an X-ray tube configured to generate X-rays and a collimator configured to guide the generated X-rays toward the object. The object may be a living body of the human being or animal but is not limited thereto. Anything, an internal structure of which can be imaged by the X-ray imaging apparatus 100, may be used as the object.

The operator 125 configured to provide a user interface may be disposed at one side of the X-ray source 120. A user who performs diagnosis of the object using the X-ray imaging apparatus 100 may be a medical team including a doctor, a radiologist, and a nurse. However, the present disclosure is not limited thereto, and anyone who uses the X-ray imaging apparatus 100 may be the user.

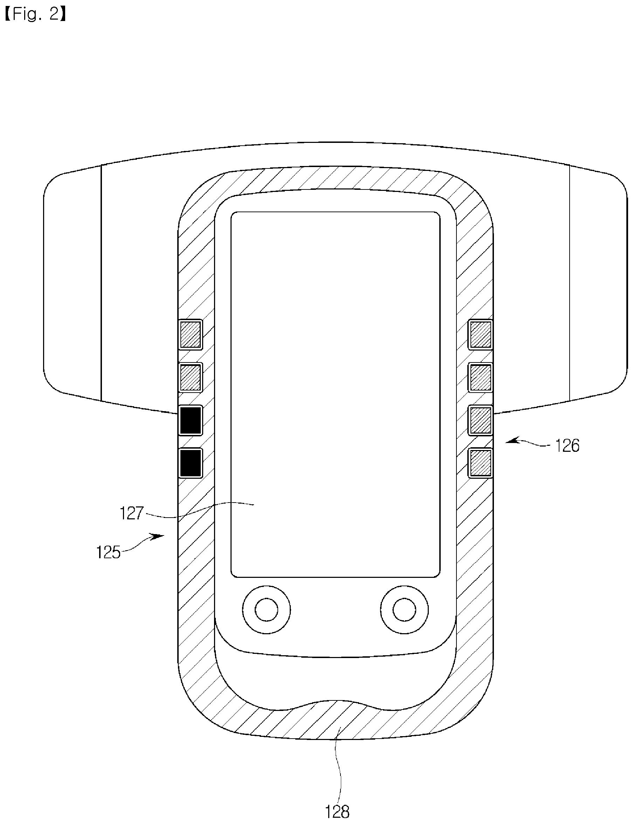

FIG. 2 is a perspective view illustrating the operator 125 of the X-ray imaging apparatus 100.

Referring to FIG. 2, the operator 125 may include a button 126 and a display panel 127. A user may press the button 126 or touch the display panel 127 to input a variety of information about the X-ray imaging or to operate a variety of devices. For example, the user may input a movement direction and a movement position of the x-ray source 120 through the button 126 or the display panel 127.

The display panel 127 may be implemented by a cathode ray tube (CRT), a digital light source processing (DLP) panel, a plasma display panel, a liquid crystal display (LCD) panel, an electro luminescence (EL) panel, an electrophoretic display (EPD) panel, an electrochromic display (ECD) panel, a light emitting diode (LED) panel or an organic light emitting diode (OLED) panel, but is not limited thereto.

The operator 125 may include a central processing unit (CPU) implemented by a micro-processor, a graphic processing unit (GPU), and a variety of storage device, and those devices may be provided in a printed circuit board (PCB) built in therein. Since the operator 125 includes a printed circuit board and is provided in one side of the X-ray source 120, the operator 125 may be referred to as "tube head board" or "THU".

The operator 125 may include a handle 128 so that a user can grasp. That is, the user may grasp the handle 128 of the operator 125 and may apply a force or torque to the X-ray source 120 so as to move the X-ray source 120. In FIG. 2, the handle 128 is disposed at a lower portion of the operator 125. However, the position of the handle 128 is not limited thereto and thus the handle 128 may also be disposed in a different position of the operator 125.

The workstation 170 includes a user interface portion 173, and the workstation 170 together with the operator 125 provides a user interface. The user interface portion 173 may include an input 171 and a display 172 to receive a user command for X-ray imaging and to display a variety of information about X-ray imaging. For example, the user may set a condition for X-ray imaging according to an image capturing position or input a movement command of the x-ray source 120 or an X-ray imaging start command through the user interface portion 173. By using the user interface portion 173, the user may check an image acquired by the X-ray imaging.

The input 171 may include a hardware type input device such as a variety of button, a switch, a keyboard, a mouse, a track ball, various levers, a handle and a stick. According to embodiments, when the input 171 is implemented by a foot switch or a foot pedal, the input 171 may be provided in a lower portion of the body 101.

The input 171 may include a graphical user interface (GUI), i.e., software type input device such as a touch screen, to receive a user input. A touch pad may be implemented by touch screen panel (TSP) and have a mutual layer structure with the display 172.

In the same as the display panel 127 of the operator 125, the display panel 172 may be implemented by a cathode ray tube (CRT), a digital light source processing (DLP) panel, a plasma display panel, a liquid crystal display (LCD) panel, an electro luminescence (EL) panel, an electrophoretic display (EPD) panel, an electrochromic display (ECD) panel, a light emitting diode (LED) panel or an organic light emitting diode (OLED) panel, but is not limited thereto.

As mentioned above, when the display 172 is formed with a touch screen panel (TSP) that make mutual layer structure with the touch pad, the display 172 may also be used as the input device other than the display device.

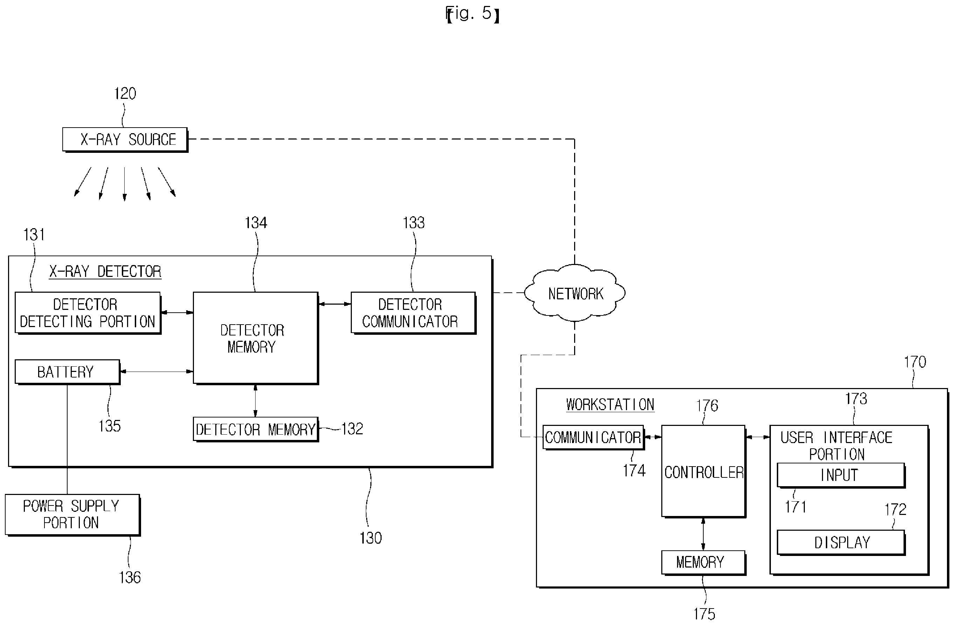

Various processing units, such as a central processing unit (CPU) or a graphic processing unit (GPU), and a printed circuit board (PCB) may be built in the workstation 170, and various kinds of storing units may also be built in the workstation 170, as needed. Thus, a main component of the X-ray imaging apparatus 100, e.g., a controller 176 (refer to FIG. 5) may be disposed in the workstation 170 so as to perform various determinations for the operation of the x-ray imaging apparatus 100 or to generate various control signals.

The X-ray detector 130 is an apparatus that detects X-rays transmitted through the object. An incidence surface on which the X-rays transmitted through the object are incident, may be provided on a front surface of the X-ray detector 130, and a detection panel configured to detect the incident X-rays may disposed in the inside of the X-ray detector 130. A plurality of pixels responding to the X-rays may be arranged on the detection panel to have a matrix shape. A handle may be disposed in the center of an upper end of the X-ray detector 130 so that the user may easily move or carry the X-ray detector 130.

In the outside or inside of the X-ray detector 130, a detecting portion 131 configured to detect information about storage position of the X-ray detector 130 is provided. Hereinafter, the storage position information may mean position information of the detector storage portion 150 in which the X-ray detector 130 is stored. The detecting portion 131 may employ an acceleration sensor or a magnetic sensor. The X-ray detector 130 may collect its storage position information via the detecting portion 131.

In the inside of the X-ray detector 130, a battery 135 is provided for the operation of the X-ray detector 130, wherein the battery 135 may include a rechargeable secondary battery.

The X-ray detector 130 may be stored in one of a plurality of detector storage portions 150:151 and 152 provided in the body 101 of the X-ray imaging apparatus 100. Particularly, the detector storage portion 150 may include a first detector storage portion 151 and a second detector storage portion 152 disposed adjacent to the first detector storage portion 151. According to embodiments, the detector storage portion 150 may further include a third detector storage portion in addition to the first detector storage portion 151 and the second detector storage portion 152. Hereinafter for convenience of description, a case in which the detector storage portion 150 is divided into the first detector storage portion 151 and the second detector storage portion 152 will be described as an example.

According to the present disclosure, the X-ray imaging apparatus 100 is provided with the plurality of detector storage portions 150: 151 and 152 and thus in order to display information about the X-ray detector 130 (e.g., charging information of the X-ray detector 130) on the display 172 of the workstation 170, it may be required to precede a process to determine which X-ray detector 130 is stored in which detector storage portion 150.

According to an embodiment, the X-ray imaging apparatus 100 may collect the storage position information together with the charging information of the X-ray detector 130 from the x-ray detector 130, and display the information on the display172 of the workstation 170. Since it is required to display the charging information of the X-ray detector 130 stored in the detector storage portion 150 in order to precisely provide the charging information of the X-ray detector 130 to a user, it may be required to determine which X-ray detector 130 is stored in which detector storage portion 150.

According to an embodiment, the X-ray imaging apparatus 100 may design the detector storage portion 150 in various ways and allow the X-ray detector 130 to collect its storage position information.

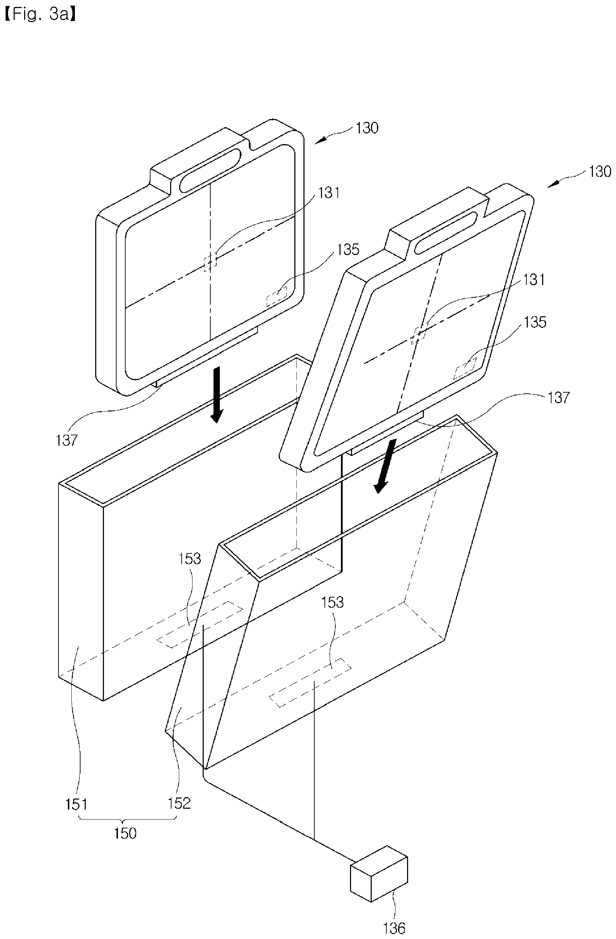

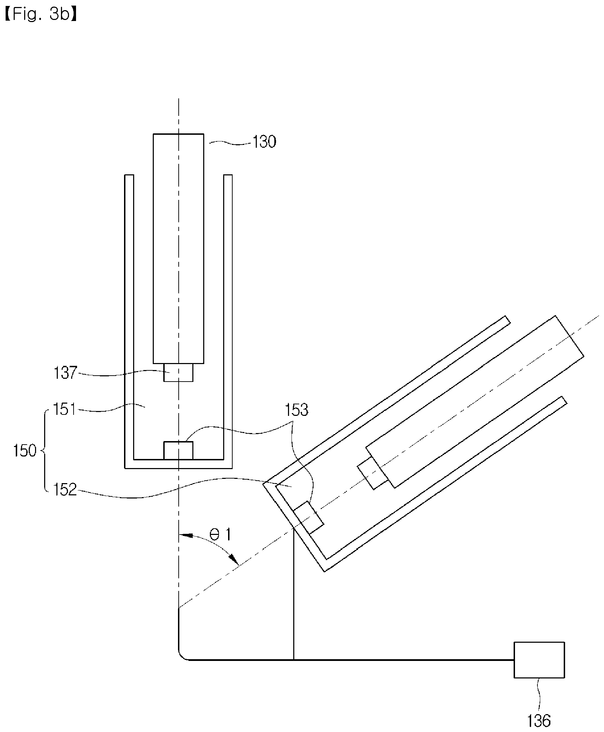

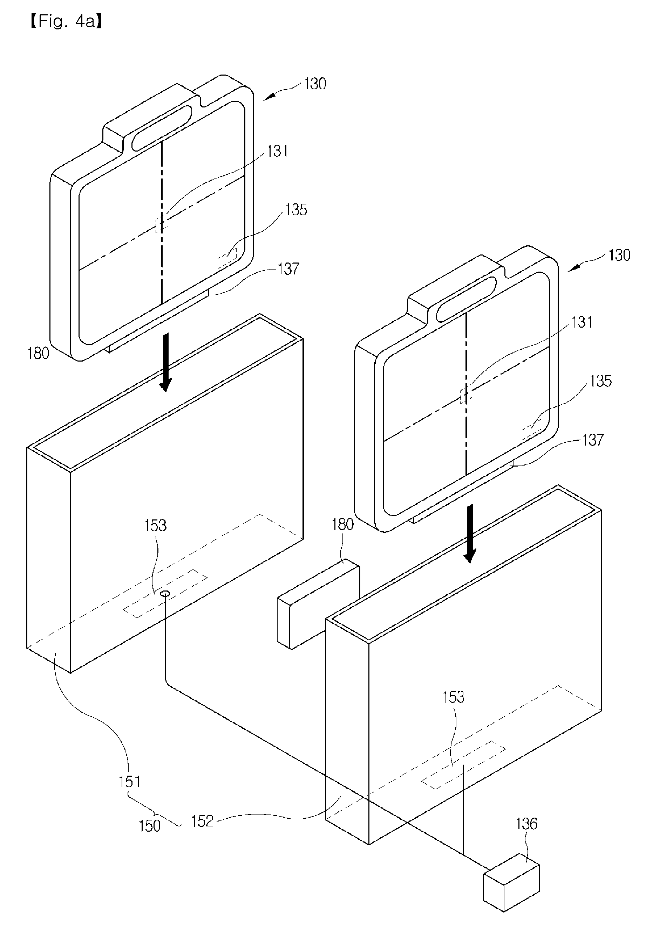

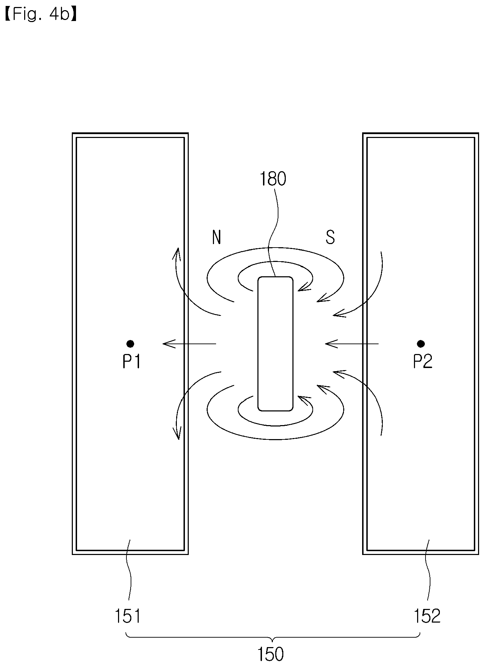

FIGS. 3 and 4 are views illustrating examples of the detector storage portion 150 of the X-ray imaging apparatus 100.

FIG. 3A is a perspective view illustrating the detector storage portion 150 in accordance with an embodiment and FIG. 3B is a cross-sectional view illustrating the detector storage portion 150 in accordance with an embodiment.

Referring to FIGS. 3A and 3B, according to an embodiment, the detector storage portion 150 of the X-ray imaging apparatus 100 may be provided such that the first detector storage portion 151 and the second detector storage portion 152 are inclined at a first angle (Θ1). Particularly, the first detector storage portion 151 and the second detector storage portion 152 may be inclined such that an angle between a center axis of the first detector storage portion 151 and a center axis of the second detector storage portion 152 becomes a first predetermined angle (Θ1). The first angle (Θ1) may be approximately 3 to 10 degrees, but the range of the first angle (Θ1) is not limited thereto.

A power supply portion 136 configured to supply power to the X-ray detector 130 may be installed in the first and second detector storage portion 151 and 152. When the X-ray detector 130 is stored in the detector storage portion 150, the X-ray detector 130 may be connected to a connection port 153 provided in the detector storage portion 150 via a connection port 137 provided in a bottom surface of the X-ray detector 130, and thus the X-ray detector 130 may be supplied with the power.

In a state in which the battery 135 of the X-ray detector 130 is required to be charged, when the X-ray detector 130 is stored in the detector storage portion 150, the battery 135 of the X-ray detector 130 may be charged through the power supply portion 136, and charging information of the battery 135 of the X-ray detector 130 may be displayed on the display 172 of the workstation 170.

As illustrated in FIGS. 3A and 3B, when the X-ray detector 130 to which the acceleration sensor is installed, is stored in the detector storage portion 150, an output value of the acceleration sensor may vary according to that the X-ray detector 130 is stored in which detector storage portion 150.

Particularly, according to an embodiment, a three-axis acceleration sensor may be applied to the detecting portion 131 of the X-ray detector 130 and the three-axis acceleration sensor may detect an acceleration in the X, Y, and Z axis directions when moving in the three dimensions. Since the acceleration sensor typically detects the acceleration of gravity in a stopped state, the acceleration sensor may output -g value (negative g value) in the Z axis direction, and thus the acceleration sensor may detect an inclined angle of the X-ray detector 130 based on the above mentioned principle.

Therefore, when the inclination angles of the first detector storage portion 151 and the second detector storage portion 152 are different from each other, a gradient value detected by the acceleration sensor may vary according to that the X-ray detector 130 is stored in which detector storage portion 150. The X-ray imaging apparatus 100 may detect a storage position of the X-ray detector 130 based on the gradient information collected from the acceleration sensor of the X-ray detector 130.

The X-ray detector 130 may transmit the gradient information to the workstation 170 in response to a request from a controller 176 (refer to FIG. 5) described later to provide the gradient information to a process of determining the storage position of the X-ray detector 130.

FIG. 4A is a perspective view illustrating a detector storage portion 150 according to another example of an embodiment, and FIG. 4B is a cross-sectional view illustrating the detector storage portion 150 according to another example of an embodiment.

Referring to FIGS. 4A and 4B, according to another embodiment, as for the detector storage portion 150 of the X-ray imaging apparatus 100, a magnet 180 may be disposed between the first detector storage portion 151 and the second detector storage portion 152 of the detector storage portion 150. According to embodiments, the magnet 180 may be disposed between the first detector storage portion 151 and the second detector storage portion 152 while the first detector storage portion 151 and the second detector storage portion 152 are inclined at a predetermined angle. For convenience of description, a description the same as those in FIG. 3 will be omitted and thus a description of the power supply portion 136 will be omitted.

As illustrated in FIG. 4, when the X-ray detector 130 having a magnetic sensor is stored in the detector storage portion 150, an output value of the magnetic sensor may vary according to that the X-ray detector 130 is stored in which detector storage portion 150.

Particularly, as illustrated in FIGS. 4A and 4B, when the magnet 180 is disposed, a direction of a magnetic flux around the first detector storage portion 151 and a direction of a magnetic flux around the second detector storage portion 152 may be different from each other due to the general characteristics of the magnet 180. In other words, since the direction of the lines of magnetic force is from the N pole to the S pole, the direction of the magnetic flux formed in an inner space (P1) of the first detector storage portion 151 that is adjacent to the N pole may be different from the direction of the magnetic flux formed in an inner space (P2) of the second detector storage portion 152 that is adjacent to the S pole.

The X-ray detector 130 may collect magnetic flux density information around the magnetic sensor installed inside thereof. The X-ray detector 130 may transmit the magnetic flux density information to the workstation 170 in response to a request from the controller 176 of the workstation 170 (refer to FIG. 5) to provide the magnetic flux density information to the process of determining the storage position of the X-ray detector 130

Hereinbefore the formation of the detector storage portion 150 has been described.

Hereinafter components of the X-ray imaging apparatus 100 capable of determining which X-ray detector 130 is stored in which detector storage portion 150, and functions of components, will be described in detail with reference to a given control block diagram.

FIG. 5 is a control block diagram illustrating the X-ray imaging apparatus 100 in accordance with an embodiment.

Referring to FIG. 5, according to an embodiment, the X-ray imaging apparatus 100 may include the X-ray source 120, the X-ray detector 130, and the workstation 170. The X-ray source 120 and the X-ray detector 130 may be connected to the workstation 170 via a wired or wireless network.

The X-ray source 120 is an apparatus that generates X-rays and irradiates the X-rays to the object. The X-rays generated from the X-ray source 120 passes through the object and is detected by the X-ray detector 130.

The X-ray detector 130 is an apparatus that detects X-rays, which are irradiated from the X-ray source 120 and then transmitted through an object. Detection of the X-rays is performed by the detection panel inside the X-ray detector 130. In addition, the detection panel converts the detected X-rays into electrical signals to acquire X-ray images of the inside of the object.

The X-ray detector 130 may include the detecting portion 131, the memory 132, the communicator 133, and the controller 134. In addition, the X-ray detector 130 may include the battery 135 to supply the power to each component of the X-ray detector 130. The battery 135 may be supplied with the power through the power supply portion 136 connected to the X-ray detector 130. Hereinafter, the memory 132, the communicator 133, and the controller 134 will be referred to as the detector memory 132, the detector communicator 133, and the detector controller 134 to be distinguished from components provided in the workstation 170 described later.

The detecting portion 131 may include at least one sensor to collect the storage position information of the X-ray detector 130. The detecting portion 131 may be provided on the rear surface or the side surface of the X-ray detector 130 or may be provided inside the X-ray detector 130. That is, the detecting portion 131 may be provided anywhere on the X-ray detector 130 as long as it does not affect the detection of the X-ray detector 130.

The detecting portion 131 may include an acceleration sensor and a magnetic sensor, but is not limited thereto. As long as capable of detecting the position of the X-ray detector 130, the detecting portion 131 may employ a different type of sensor.

The detector memory 132 temporarily or non-temporarily stores data and programs for operation of the X-ray detector 130.

The detector memory 132 may store a sensor value, which is outputted from the detecting portion 131 when the X-ray detector 130 is stored in the detector storage portion 150. According to an example, the detector memory 132 may store the gradient information of the X-ray detector 130 detected by the acceleration sensor. In addition, the detector memory 132 may store magnetic flux direction information detected by the magnetic sensor.

The sensor value stored in the detector memory 132 may be provided to the controller 176 at a request of the controller 176 of the workstation 170. The controller 176 may determine a position to which the X-ray detector 130 is mounted, i.e., a mounting position of the X-ray detector 130, based on the received sensor value.

The detector memory 132 may store identification (ID) information assigned to the X-ray detector 130. When an identification (ID) of the X-ray detector 130 is changed, the detector memory 132 may store the changed ID information. The controller 176, described later, may determine which X-ray detector 130 is stored in which detector storage portion 150 based on the ID information.

The detector memory 132 may include at least one of storage medium among flash memory type, a hard disk type, a multimedia card micro type, a card type memory (e.g., SD or XD memory, etc.), a random access memory (RAM), a static random access memory (SRAM), a read-only memory (ROM), an electrically erasable programmable read-only memory (EEPROM), a programmable read-only memory (PROM), a magnetic memory, a magnetic disc, or an optical disc, but is not limited thereto. The detector memory 132 may be implemented by well-known technology by those skilled in the art.

The detector communicator 133 transmits and receives various signals and data to and from the workstation 170 via a wired and/or wireless communication.

The detector communicator 133 may transmit a sensor value output from the detecting portion 131 to the workstation 170. The detector communicator 133 may transmit the ID information of the X-ray detector 130 to the workstation 170, and according embodiments, the detector communicator 133 may receive the ID information assigned to the X-ray detector 130, from the workstation 170. As mentioned above, the assigned ID information may be changed and in this case, the detector communicator 133 may receive the changed ID information from the workstation 170.

The detector communicator 133 may include the connection port 137. The connection port 137 of the detector communicator 133 may be connected to the connection port 153 of the detector storage portion 150 to enable the communication of the X-ray detector 130 and the workstation 170. That is, the connection port 137 and 153 connecting the X-ray detector 130 to the detector storage portion 150 may function as the detector communicator 133.

According to embodiments, the detector communicator 133 may include a variety of communication module such as a wireless internet module, a short range communication module, and a mobile communication module.

The wireless internet module represents a module capable of performing a communication connected to an external network according to the communication protocol such as wireless LAN (WLAN), Wi-Fi, wireless broadband (Wibro), World Interoperability for Microwave Access (Wimax), or High Speed Downlink Packet Access (HSDPA).

The short range communication module represents a module capable of performing a communication with an external device placed in the short range according to the short range communication method such as Bluetooth, Radio Frequency Identification (RFID), infrared Data Association (IrDA), Ultra Wideband (UWB), or ZigBee.

The mobile communication module represents a module capable of performing a communication by connecting to a mobile communication network according to a variety of mobile communication protocol such as 3rd Generation (3G), 3rd Generation Partnership Project (3GPP), or Long Term Evolution (LTE).

However, an example of a communication module is not limited thereto. Other types of communication modules may be employed if they are capable of communicating with the workstation 170.

The detector controller 134 controls the overall operation of the X-ray detector 130. The detector controller 134 may control the respective structures of the X-ray detector 130, i.e., the detecting portion 131, the detector memory 132 and the detector communicator 133. The detector controller 134 may be various processors including at least one chip in which a direct circuit is formed.

The workstation 170 may include a user interface portion 173, a communicator 174, a memory 175, and a controller 176.

The user interface portion173 may include an input 171 and a display 172 to receive user commands for X-ray imaging or to display various information related to X-ray imaging.

The display 172 may display information about the X-ray detector 130 stored in the detector storage portion 150, e.g., the charging information of the X-ray detector 130. The charging information of the X-ray detector 130 may include information about a remaining amount of the battery 135 of the X-ray detector 130.

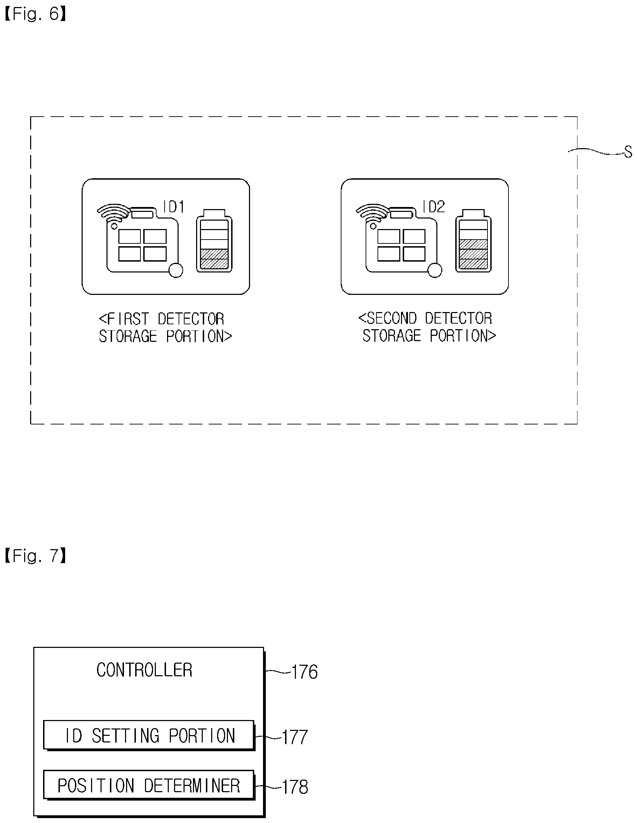

FIG. 6 is a view illustrating an example of a screen (S) of the display displaying charge information of the X-ray detector 130.

Referring to FIG. 6, the screen (S) of the display 172 may display remaining battery information for each of the plurality of detector storage portion 150: 151 and 152. According to an example, a phrase for distinguishing the detector storage portion 150 may be displayed at the bottom of the screen (S), and an icon of the X-ray detector 130 stored in the detector storage portion 150 and a battery icon of the X-ray detector 130 may be displayed in an upper side of the phrase. The ID information of the X-ray detector 130 may be displayed around the icon of the X-ray detector 130, and the battery icon may display the remaining battery information. FIG. 6 is merely an example of a screen to display the charging information of the X-ray detector 130, but the configuration of the screen for displaying the charging information of the X-ray detector 130 is not limited to that shown in FIG. 6.

The communicator 174 may receive the ID information and the storage position information of the X-ray detector 130 from the x-ray detector 130. The communicator 174 may receive the charging information of the X-ray detector 130 from the X-ray detector 130. The communicator 174 may receive the ID information, the storage position information and the charging information of the x-ray detector 130 at the same time, or may receive them in sequence.

The communicator 174 transmits the received information to the controller 176, and the controller 176 determines which X-ray detector 130 is stored in which detector storage portion 150, based on the ID information and storage position information that is received from the communicator 174.

The memory 175 temporarily or non-temporarily store data and programs for operation of the X-ray imaging apparatus 100.

The memory 175 may pre-store the sensor value of the detecting portion 131 that is changed according to the storage position of the X-ray detector 130. When the detecting portion 131 includes an acceleration sensor, the memory 175 may pre-store the gradient information that is changed according to the storage position of the X-ray detector 130. When the detecting portion 131 includes a magnetic sensor, the memory 175 may pre-store the magnetic flux direction information that is changed according to the storage position of the X-ray detector 130.

The memory 175 may pre-store the ID information of the X-ray detector 130 that is set according to the use. That is, the memory 175 may pre-store table ID information, stand ID information, and portable ID information set according to the purpose.

The memory 175 may store a program for determining which X-ray detector 130 is stored in which detector storage portion 150 based on the ID information and storage position information. The memory 175 may store a program for setting an ID of the X-ray detector 130 according to the purpose, a program for allocating an ID to the X-ray detector 130 and a program for changing the ID assigned to the X-ray detector 130.

The memory 175 may be implemented in the same manner as the aforementioned detector memory 132, and hereinafter a description the same as the above mentioned description will be omitted.

The controller 176 controls the overall operation of the workstation 170. The controller 176 may control each component of the workstation 170, such as the user interface portion173, the communicator 174 and the memory 175.

The controller 176 may assign or change the ID of the x-ray detector 130. The controller 176 may receive the ID information and storage position information of the X-ray detector 130 from the X-ray detector 130, determine a storage position of the X-ray detector 130 based on the ID information and storage position information and identify the stored X-ray detector 130. The ID information may include Internet Protocol (IP) address, and Media Access Control (MAC) address, but is not limited thereto. Any type thereof may be applicable as long as it can identify the X-ray detector 130.

The controller 176 may be a variety of processors that includes at least one chip in which an integrated circuit is formed. The controller 176 may be provided on a single processor, or alternatively may be provided separately to a plurality of processors.

FIG. 7 is a view illustrating a detailed configuration of the controller 176 according to FIG 5.

Referring FIG. 7, the controller 176 may include an ID setting portion177 and a position determiner 178.

The ID setting portion177 may set an ID of the X-ray detector 130 according to the usage of the X-ray detector 130. The ID setting portion177 may set an ID for table, an ID for stand and an ID for portable usage. The ID setting portion177 may set an ID based user's input through the user interface portion 173, or alternatively the ID setting portion177 may automatically set an ID in a systematic manner.

The ID that is set by the ID setting portion 177 such as the ID for table, the ID for stand and the ID for portable usage may be stored in the memory 175.

The position determiner 178 may determine which X-ray detector 130 is stored in which detector storage portion 150 based on the ID information and storage position information of the X-ray detector 130.

Particularly, the position determiner 178 may determine a storage position of the X-ray detector 130 based on the gradient information of the X-ray detector 130 detected by the acceleration sensor. According to embodiments, when the X-ray imaging apparatus 100 passes a ramp, the position determiner 178 may determine a storage position of the X-ray detector 130 by applying a gradient of a ramp together with the gradient information of the X-ray detector 130 detected by the acceleration sensor. Meanwhile, the position determiner 178 may determine a storage position of the X-ray detector 130 based on the magnetic flux direction information detected by the magnetic sensor. A detail description thereof will be described later with reference to a description of a control process of the X-ray imaging apparatus 100.

Hereinbefore the X-ray detector 130 and the X-ray imaging apparatus 100 including the same according to one embodiment have been described.

Next, various embodiments of the X-ray imaging apparatus 100 will be described.

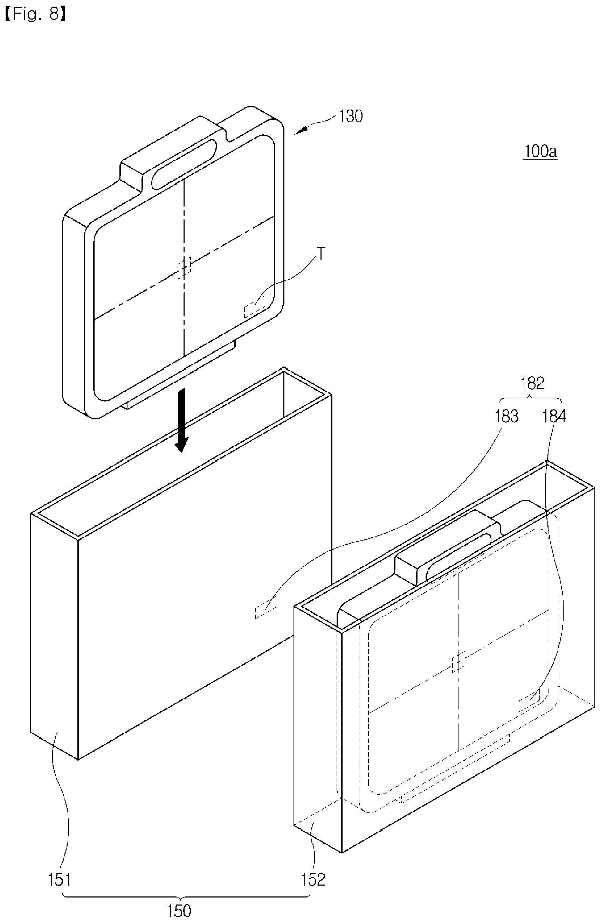

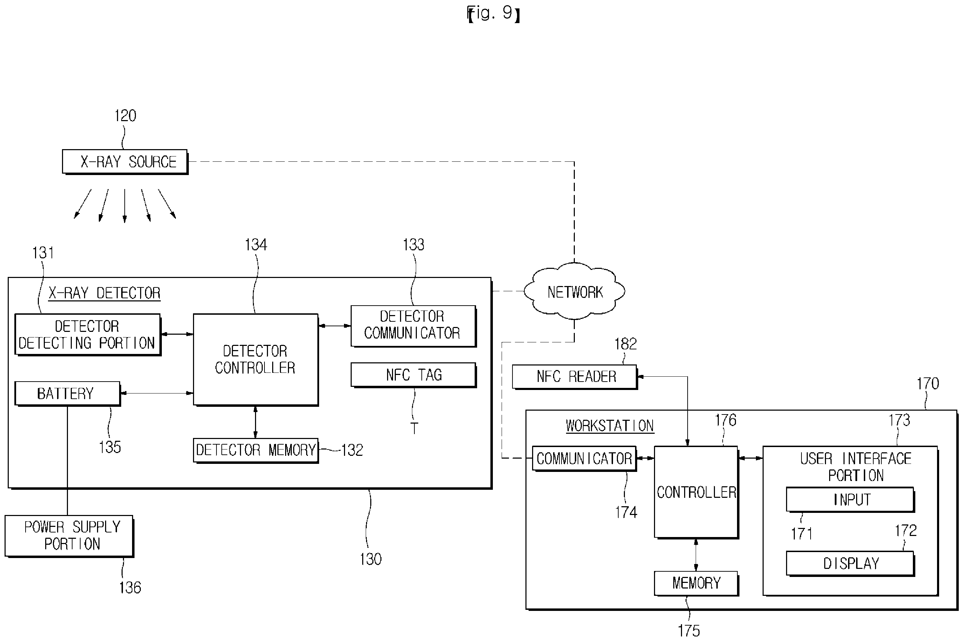

FIG. 8 is a view illustrating an example of a detector storage portion of the X-ray imaging apparatus according to another embodiment, and FIG. 9 is a control block diagram of the X-ray imaging apparatus of FIG. 8.

Referring to FIG. 8, according to another embodiment, Near Field Communication (NFC) tag (T) may be attached on one side of an X-ray detector 130 of an X-ray imaging apparatus 100a and a Near Field Communication (NFC) reader, 182 may be installed inside of the detector storage portion 150.

The detector storage portion 150 may include the first detector storage portion 151 and the second detector storage portion 152, and the NFC reader 182 may be installed in an inner surface of the first detector storage portion 151 and the second detector storage portion 152, respectively. Hereinafter a NFC reader 182 installed in an inner surface of the first detector storage portion 151 is defined as a first NFC reader 183, and a NFC reader 182 installed in an inner surface of the second detector storage portion 152 is defined as a second NFC reader 184.

Hereinafter according to another embodiment, components of the X-ray imaging apparatus 100a, and functions of components, will be described in detail.

Referring to FIG. 9, according to another embodiment, the X-ray imaging apparatus 100a may include an X-ray source 120, an X-ray detector 130, a NFC reader 182 and a workstation 170. The X-ray source 120, the X-ray detector 130 and the NFC reader 182 may be connected to the workstation 170 via a wired and/or wireless communication network. Hereinafter a description about differences from FIG. 5 will be mainly described.

The X-ray detector 130 may include the detector memory 132, the detector communicator 133 and the detector controller 134. According to embodiments, the X-ray detector 130 may further include the battery 135, and the battery 135 may be supplied with the power from the power supply portion 136. Hereinafter a description the same as the above mentioned description will be omitted.

A Near Field Communication (NFC) tag (T) may be attached on one side of an X-ray detector 130. ID information of the X-ray detector 130 may be stored in the NFC tag (T). The ID information of the X-ray detector 130 may be provided to the process in which the controller 176 determines a storage position of the X-ray detector 130.

When the X-ray detector 130 is stored in the detector storage portion 150, the NFC reader 182 may recognize a NFC tag (T), and transmit the recognized information to the workstation 170.

The workstation 170 may include the user interface portion 173, the communicator 174, the memory 175 and the controller 176. Hereinafter a description the same as the above mentioned the user interface portion 173 and the communicator 174 will be omitted.

The memory 175 may store position information of the NFC reader 182. For example, the memory 175 may store position information of the first and second NFC reader 182: 183 and 184. The memory 175 may transmit the position information of the first and second NFC reader 182: 183 and 184 to the controller 176 at the request of the controller 176, and the position information may be provided to the process in which the controller 176 determines the storage position of the X-ray detector 130.

The controller 176 may determine a storage position of the X-ray detector 130 based on the NFC tag (T) information collected from the NFC reader 182 and the position information of the NFC reader 182 stored in the memory 175. For example, the controller 176 may determine that ID information of the X-ray detector 130 collected from the first NFC reader 183 corresponds to ID information of the X-ray detector 130 stored in the first detector storage portion 151. In the same manner, the controller 176 may determine that ID information of the X-ray detector 130 collected from the second NFC reader 184 corresponds to ID information of the X-ray detector 130 stored in the second detector storage portion 152.

Therefore, when receiving the ID information of the X-ray detector 130 from the first NFC reader 183, the controller 176 may determine that the X-ray detector 130 having the corresponding ID information is stored in the first detector storage portion 151. In the same manner, when receiving the ID information of the X-ray detector 130 from the second NFC reader 184, the controller 176 may determine that the X-ray detector 130 having the corresponding ID information is stored in the second detector storage portion 152.

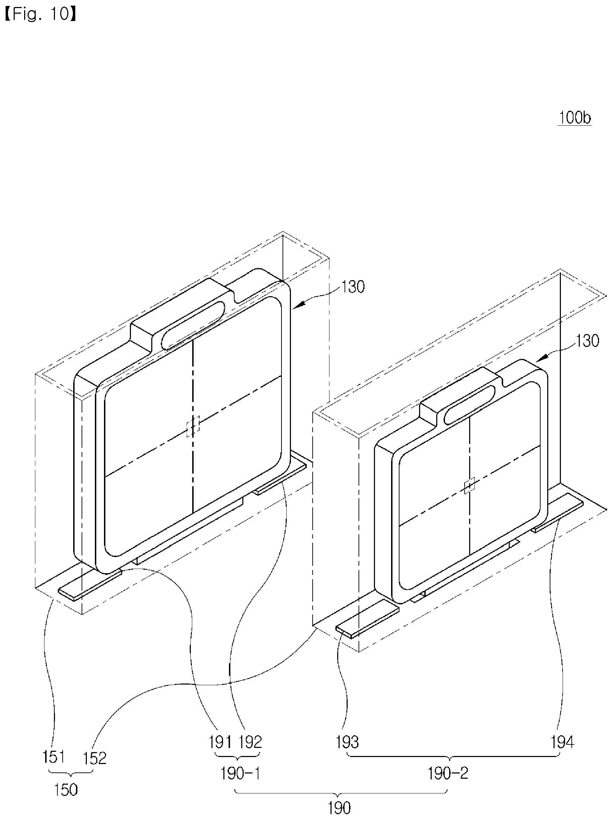

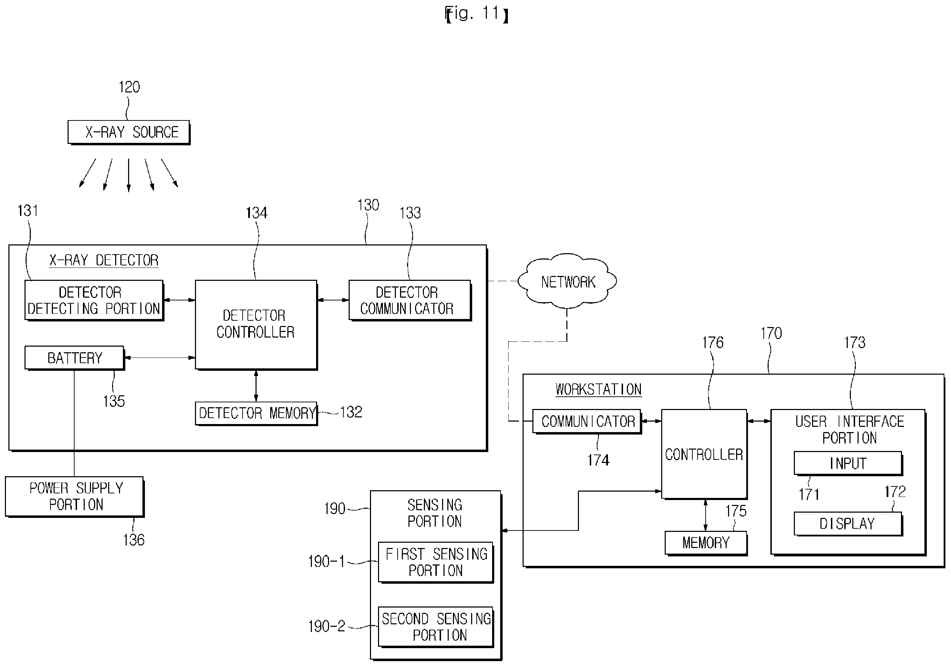

FIG. 10 is a view illustrating an example of a detector storage portion of the X-ray imaging apparatus according to another embodiment, and FIG. 11 is a control block diagram of the X-ray imaging apparatus of FIG. 10.

Referring to FIG. 10, according to another embodiment, a detector storage portion 150 of an X-ray imaging apparatus 100b may include a first detector storage portion 151 and a second detector storage portion 152 adjacent to the first detector storage portion 151. A sensing portion 190 configured to detect the size of the X-ray detector 130 stored in the first and second detector storage portion 151 and 152 may be installed in the first and second detector storage portion 151 and 152. Hereinafter for convenience of description, a sensing portion 190 installed in the first detector storage portion 151 may be defined as a first sensing portion 190-1 and a sensing portion 190 installed in the second detector storage portion 152 may be defined as a second sensing portion 190-2.

The first and second sensing portion 190-1 and 190-2 may include a plurality of sensors configured to detect the size of the X-ray detector 130 stored in the detector storage portion 150, and particularly, may include a photo sensor or a limit switch. However, an example of sensor applied to the first and second sensing portion 190-1 and 190-2 is not limited thereto, and thus it is to be understood that the present disclosure is to be broadly construed to include modifications within the scope of the present disclosure which are readily apparent to those skilled in the art.

The first sensing portion 190-1 may be installed in an inner surface or a bottom surface of the first detector storage portion 151. The first sensing portion 190-1 may include a 1-1 sensor 191 and a 1-2 sensor 192 separated from the 1-1 sensor 191. In the same manner, the second sensing portion 190-2 may be installed in an inner surface or a bottom surface of the second detector storage portion 152. The second sensing portion 190-2 may include a 2-1 sensor 193 and a 2-2 sensor 194 separated from the 2-1 sensor 193.

Hereinafter according to another embodiment, components of the X-ray imaging apparatus 100b, and functions of components, will be described in detail.

Referring to FIG. 11, according to another embodiment, the X-ray imaging apparatus 100b may include the x-ray source 120, the X-ray detector 130, the NFC reader 182 and the workstation 170. The x-ray source 120, the X-ray detector 130, the NFC reader 182 and the sensing portion 190 may be connected to the workstation 170 via a wired and/or wireless communication network.

The X-ray detector 130 may include the detector memory 132, the detector communicator 133 and the detector controller 134. According to embodiments, the X-ray detector 130 may further include the battery 135, and the battery 135 may be supplied with the power from the power supply portion 136. Hereinafter a description the same as the above mentioned description will be omitted

The detector memory 132 may store ID information of the X-ray detector 130. The detector memory 132 may output the ID information of the X-ray detector 130 to the controller 176 at a request of the controller 176, and the controller 176 may determine which X-ray detector 130 is stored in which detector storage portion 150 based on the ID information of the X-ray detector 130 received from the detector memory 132.

The detector memory 132 may store firmware information of the X-ray detector 130. The firmware information of the X-ray detector 130 may include size information of the X-ray detector 130. According to embodiments, the firmware information of the X-ray detector 130 may include the ID information of the X-ray detector 130. The detector memory 132 may output the size information of the X-ray detector 130 to the controller 176 at a request of the controller 176, and the controller 176 may determine which X-ray detector 130 having the corresponding size is stored in which detector storage portion 150 based on the size information of the X-ray detector 130 received from the detector memory 132.

The sensing portion 190 may be configured to collect size information of the X-ray detector 130. The sensing portion 190 may include a first and second sensing portion 190-1 and 190-2, and particularly, the first sensing portion 190-1 may include a 1-1 sensor 191 and a 1-2 sensor 192 and the second sensing portion 190-2 may include a 2-1 sensor 193 and a 2-2 sensor 194.

The sensing portion 190 may transmit sensor value information collected from the sensing portion 190 to the controller 176 at a request of the controller 176, and the sensor value information of the sensing portion 190 may be provided to determine a storage position of the X-ray detector 130.

The sensing portion 190 may collect the size information of the X-ray detector 130 in the form of sensor values as shown below. For example, when a large size X-ray detector 130 is stored in the first detector storage portion 151, a sensor value of the 1-1 sensor 191 and the 1-2 sensor 192 of the first sensing portion 190-1 may be output as an ON state. According to embodiments, when a small size X-ray detector 130 is stored in the first detector storage portion 151, a sensor value of the 1-1 sensor 191 may be output as an OFF state, and a sensor value of the 1-2 sensor 192 may be output as the ON state. Alternatively, a sensor value of the 1-1 sensor 191 may be output as the ON state, and a sensor value of the 1-2 sensor 192 may be output as the OFF state.

The workstation 170 may include the user interface portion 173, the communicator 174, the memory 175 and the controller 176. Hereinafter a description the same as the above mentioned the user interface portion 173 and the communicator 174 will be omitted.

The memory 175 may store position information of the sensing portion 190. For example, the memory 175 may store position information of the first and second sensing portion 190-1 and 190-2 and particularly the memory 175 may store the position information of the 1-1 sensor 191, the 1-2 sensor 192, the 2-1 sensor 193, and the 2-2 sensor 194. The memory 175 may transmit the position information of the first and second sensing portion 190-1 and 190-2 to the controller 176 at the request of the controller 176, and the position information may be provided to the process of determining the storage position of the X-ray detector 130.

The memory 175 may store the sensor value information transmitted from the sensing portion 190. For example, the memory 175 may store sensor value information of the first and second sensing portion 190-1 and 190-2, and particularly, the memory 175 may store sensor value information of the 1-1 sensor 191, the 1-2 sensor 192, the 2-1 sensor 193, and the 2-2 sensor 194. The memory 175 may transmit the sensor value information of the first and second sensing portion 190-1 and 190-2 to the controller 176 at a request of the controller 176, and the sensor value information may be provided to determine a storage position of the X-ray detector 130.

The controller 176 may control the overall operation of the workstation 170 and control each component of the workstation 170.

The controller 176 may determine a storage position of the X-ray detector 130 based on the sensor value information collected from the sensing portion 190, the position information of the sensing portion 190 stored in the memory 175, and the ID information and the firmware information of the X-ray detector 130 collected from the detector memory 132.

For example, the controller 176 may determine that the sensor value information collected from the first sensing portion 190-1 corresponds to the size information of the X-ray detector 130 stored in the first detector storage portion 151. In the same manner, the controller 176 may determine that the sensor value information collected from the second sensing portion 190-2 corresponds to the size information of the X-ray detector 130 stored in the second detector storage portion 152.

The controller 176 may collect the firmware information of the X-ray detector 130 from the X-ray detector 130 and then match the size information of the X-ray detector 130 contained in the firmware information, with the size information of the X-ray detector 130 collected from the sensing portion 190.

When the size information of the X-ray detector 130 contained in the firmware information coincides with the size information of the X-ray detector 130 collected from the first sensing portion 190-1, the controller 176 may determine that the corresponding X-ray detector 130 is stored in the first detector storage portion 151. In the same manner, when the size information of the X-ray detector 130 contained in the firmware information coincides with the size information of the X-ray detector 130 collected from the second sensing portion 190-2, the controller 176 may determine that the corresponding X-ray detector 130 is stored in the second detector storage portion 152.

According to the present disclosure, the X-ray detector 130 may be stored not only in the detector storage portion 150 of the mobile X-ray imaging apparatus 100, but also in a stand mounting portion or a table mounting portion of the X-ray imaging apparatus 100, which are provided in a ceiling type. In addition, a determination method of the storage position of the X-ray detector 130, which is similar with the above mentioned method, may be applied thereto. Hereinafter the X-ray imaging apparatus in the ceiling type will be described.

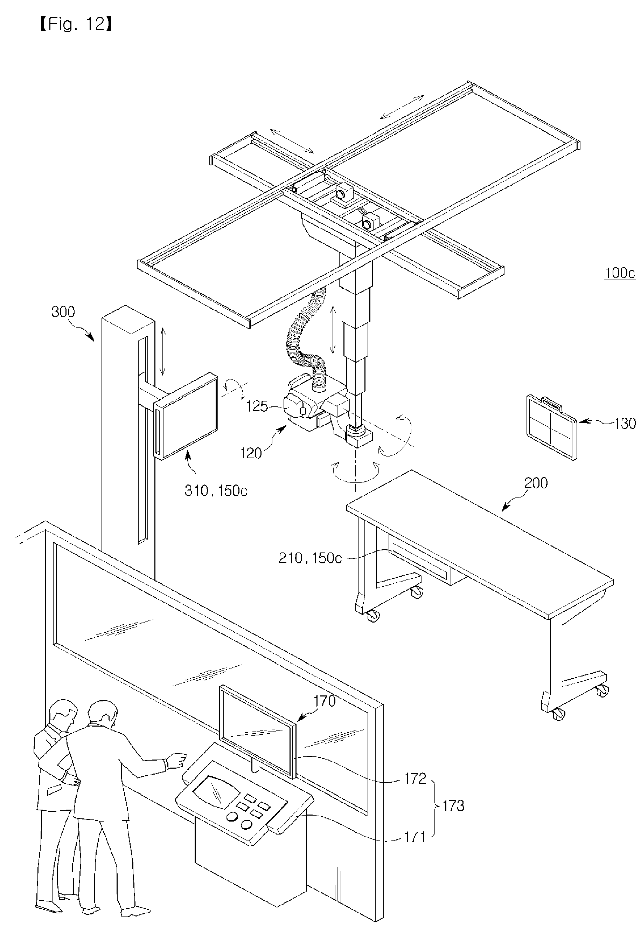

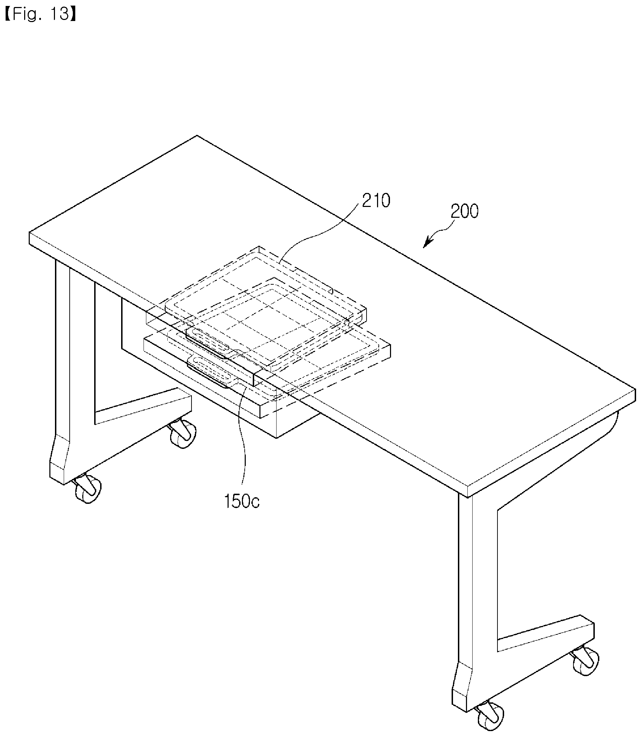

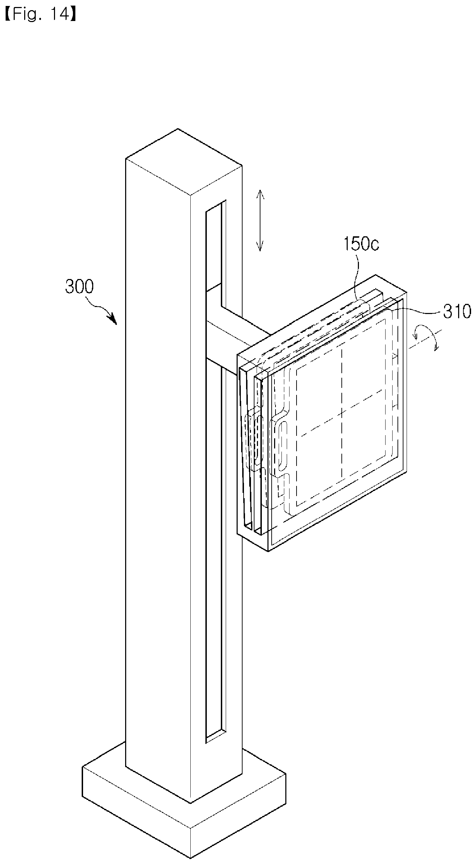

FIG. 12 is a view schematically illustrating an appearance of an X-ray imaging apparatus in the ceiling type, FIG. 13 is a view illustrating an image capturing table of the X-ray imaging apparatus of FIG. 12, and FIG. 14 is a view illustrating an image capturing stand of the X-ray imaging apparatus of FIG. 12.

Referring to FIGS. 12 to 14, according to another embodiment, an X-ray imaging apparatus 100c may include an x-ray source 120, an operator 125, an X-ray detector 130 and a workstation 170. The X-ray imaging apparatus 100c may further include an image capturing table 200 or an image capturing stand 300 to which the X-ray detector 130 is mounted or stored.

The X-ray source 120 may be an apparatus that emits X-rays to an object, and configured to be movable by a guide rail, a moving carriage and a post frame. Hereinafter a description the same as the description about the X-ray source 120 will be omitted.

The X-ray detector 130 is an apparatus that detects X-rays transmitted through an object. The X-ray detector 130 may be mounted to the image capturing table 200 or the image capturing stand 300 when performing the X-ray imaging. According to embodiments, X-ray detector 130 may be stored in the image capturing table 200 or the image capturing stand 300 when the X-ray imaging apparatus 100 is not used.

In the image capturing table 200 and the image capturing stand 300, a detector mounting portion 210 and 310 may be installed to allow the X-ray detector 130 to be installed to the image capturing table 200 and the image capturing stand 300. A detector storage portion 150c may be provided around the detector mounting portion 210 and 310 so that the X-ray detector 130 is stored when the X-ray imaging apparatus 100 is not used. Meanwhile, when the X-ray imaging apparatus is not used, the detector mounting portion 210 and 310 may be used as a detector storage portion.

Hereinafter for convenience of description, a detector mounting portion 210 provided in the image capturing table 200 may be defined as a table mounting portion 210 and a detector mounting portion 310 provided in the image capturing stand 300 may be defined as a table mounting portion 310.

The table mounting portion 210 and the detector storage portion 150c or the table mounting portion 310 and the detector storage portion 150c may be configured to allow the X-ray detector 130 to recognize its storage position. According to the embodiment, the detector mounting portion 210 and 310, and the detector storage portion 150c of the X-ray imaging apparatus 100c may be designed in the same manner as the detector storage portion 150 of the X-ray imaging apparatus 100.

Referring to FIG. 13, the table mounting portion 210 and the detector storage portion 150c may be inclined at a predetermined angle. The table mounting portion 210 may be configured to accommodate the X-ray detector 130 while being configured to allow the X-ray detector 130 to be mounted thereto upon performing the X-ray imaging, and the table mounting portion 210 may be provided in a xy plane in parallel to the ground. The detector storage portion 150c may be inclined at a predetermined angle with the ground.

Referring to FIG. 14, the table mounting portion 310 and the detector storage portion 150c may be also inclined at a predetermined angle. The table mounting portion 310 may be configured to accommodate the X-ray detector 130 while being configured to allow the X-ray detector 130 to be mounted thereto upon performing the X-ray imaging, and the table mounting portion 310 may be provided in a yz plane perpendicular to the ground. The detector storage portion 150c may be inclined at a predetermined angle with the ground.

According to embodiments, a magnet may be installed between the detector mounting portion 210 and 310 and the detector storage portion 150c. In addition, the NFC reader 182 may be provided in the detector mounting portion 210 and 310 and the detector storage portion 150c, and thus NFC tag information collected by the NFC reader 182 may be provided to a process of determining a storage position of the X-ray detector 130. Alternatively, a plurality of sensors may be installed in the detector mounting portion 210 and 310 and the detector storage portion 150c, and thus sensor value information collected by the sensor may be provided to the process of determining a storage position of the X-ray detector 130. Hereinafter a description of the method the same as the above mentioned description will be omitted.

Hereinbefore according to an embodiment, the X-ray detector 130 and the X-ray imaging apparatus 100 having the same have been described.

Hereinafter a control process of the X-ray imaging apparatus 100 will be described in detail.

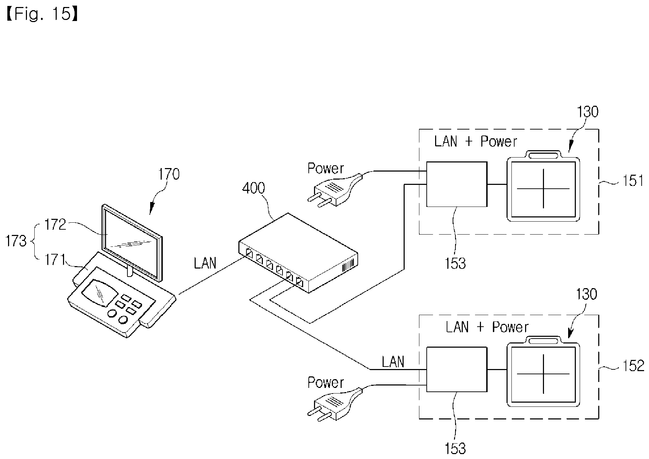

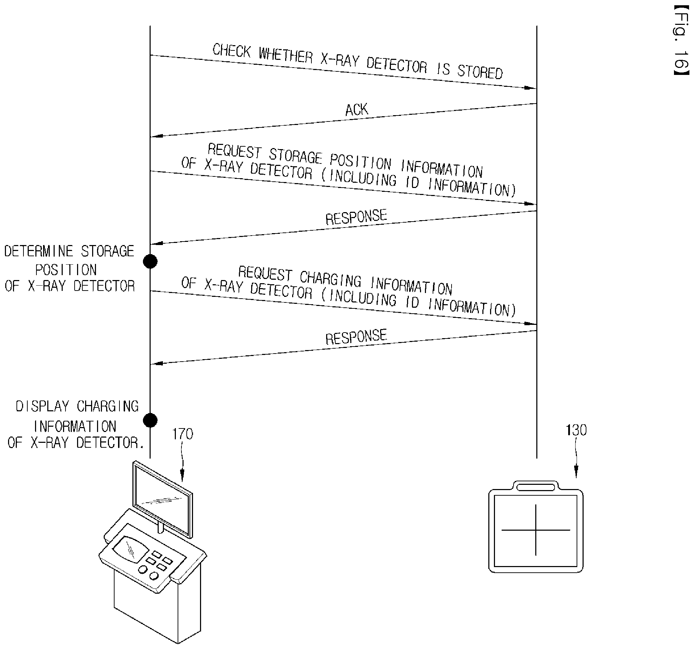

FIG. 15 is a view illustrating a connection structure between the X-ray detector 130 and the workstation 170 in accordance with an embodiment, and FIG. 16 is a view illustrating a signal flow of the X-ray detector 130 and the workstation 70 in accordance with an embodiment.

In this embodiment, in a state in which it is assumed that the X-ray detector 130 and the workstation 170 of the X-ray imaging apparatus 100, which are described in FIGS. 1 to 7, are connected as shown in FIG. 15, a signal flow of the X-ray detector 130 and the workstation 170 will be described.

Referring to FIG. 15, according to an embodiment, the workstation 170 and the X-ray detector 130 of the X-ray imaging apparatus 100 may be connected via LAN communication by using a network hub 400. The X-ray detector 130 stored in the first detector storage portion 151 and the X-ray detector 130 stored in the second detector storage portion 152 may receive the LAN communication and the power through the connection port 153 installed in the first and second detector storage portion 151 and 152. That is, the connection port 153 may be connected to the power supply portion 136 configured to supply the power to the X-ray detector 130, and then function as the communicator 174 configured to provide the LAN communication.

Referring to FIG. 16, the workstation 170 may send a ping signal to the first and second detector storage portion 151 and 152 at a predetermined time interval to determine whether the X-ray detector 130 is stored in the first and second detector storage portion 151 and 152. The workstation 170 may send a ping signal to the first and second detector storage portion 151 and 152 in a broadcast manner.

When the X-ray detector 130 is stored in the detector storage portion 150, the X-ray detector 130 may receive the ping signal from the workstation 170. When the X-ray detector 130 receives the ping, the X-ray detector 130 may send an ack signal indicating that the ping signal is received, to the workstation 170.

When the workstation 170 receives the ack signal from the X-ray detector 130, the workstation 170 may request the storage position information of the X-ray detector 130, to the X-ray detector 130. The storage position information of the X-ray detector 130 may include the sensor value information of the detecting portion 131. When the detecting portion 131 includes the acceleration sensor, the sensor value information of the detecting portion 131 may include the gradient information of the acceleration sensor. When the detecting portion 131 includes the magnetic sensor, the sensor value information of the detecting portion 131 may include the magnetic flux direction information of the acceleration sensor.

When the X-ray detector 130 receives a request for the storage position information of the X-ray detector 130, from the workstation 170, the X-ray detector 130 may send the storage position information of the X-ray detector 130 to the workstation 170. The storage position information of the X-ray detector 130 may include the ID information of the X-ray detector 130.

When the workstation 170 receives the ID information and the storage position information of the X-ray detector 130, from the X-ray detector 130, the workstation 170 may determine which X-ray detector 130 is stored in which detector storage portion 150, based on the received information.

Meanwhile, according to embodiments, the workstation 170 may additionally request the charging information of the X-ray detector 130, to the X-ray detector 130. The charging information of the X-ray detector 130 may include remaining amount information of the battery 135 of the X-ray detector 130.

When the X-ray detector 130 receives a request for the charging information of the X-ray detector 130, from the workstation 170, the X-ray detector 130 may send the charging information of the X-ray detector 130 to the workstation 170. The charging information of the X-ray detector 130 may include the ID information of the X-ray detector 130.

When the workstation 170 receives the charging information of the X-ray detector 130, from the X-ray detector 130, the workstation 170 may display the charging information of the X-ray detector 130 on the display 172 of the workstation 170, based on the received information.

As mentioned above, the workstation 170 may sequentially request the storage position information of the X-ray detector 130 and the charging information of the X-ray detector 130, to the X-ray detector 130, but alternatively, the workstation 170 may simultaneously request the storage position information of the X-ray detector 130 and the charging information of the X-ray detector 130, to the X-ray detector 130.

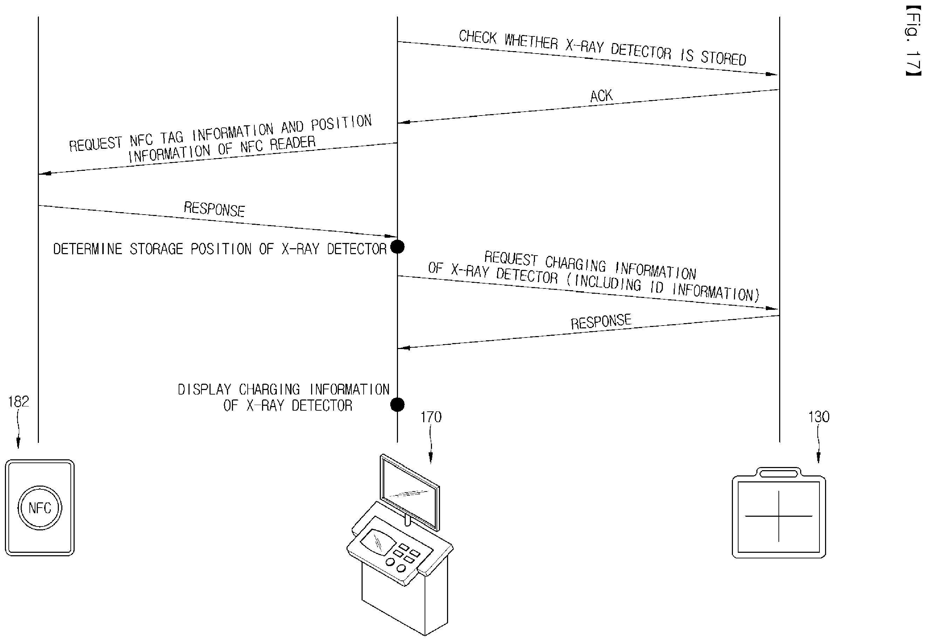

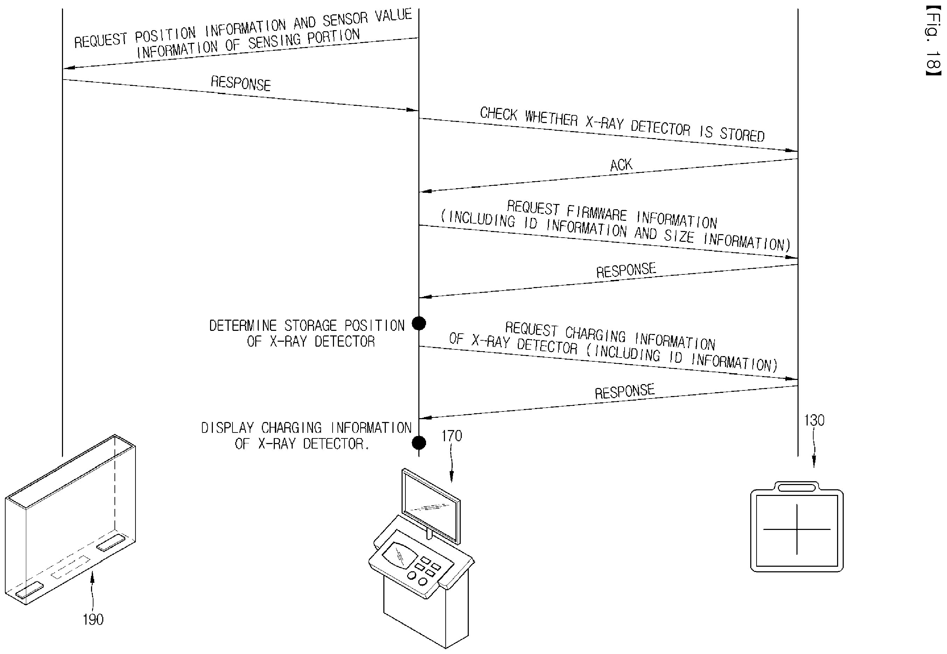

FIG. 17 is a view illustrating a signal flow of the X-ray detector 130 and the workstation 170 in accordance with another embodiment. FIG. 18 illustrates a signal flow of the X-ray imaging apparatus 100a of FIGS. 8 and 9.

Referring to FIG. 17, the workstation 170 may send a ping signal to the first and second detector storage portion 151 and 152 at a predetermined time interval to determine whether the X-ray detector 130 is stored in the first and second detector storage portion 151 and 152. The workstation 170 may send a ping signal to the first and second detector storage portion 151 and 152 in a broadcast manner.

When the X-ray detector 130 is stored in the detector storage portion 150, the X-ray detector 130 may receive the ping signal from the workstation 170. When the X-ray detector 130 receives the ping signal, the X-ray detector 130 may send an ack signal indicating that the ping signal is received, to the workstation 170.

When the workstation 170 receives the ack signal from the X-ray detector 130, the workstation 170 may request the NFC tag (T) information and the position information of the NFC reader 182, to the NFC reader 182. The NFC tag (T) may be attached to the X-ray detector 130 and thus the ID information of the X-ray detector 130 may be stored in the NFC tag (T). In addition, since the NFC reader 182 is attached to the detector storage portion 150, respectively, the position information of the NFC reader 182 may include the position information of the detector storage portion 150, i.e., the storage position information of the X-ray detector 130.

When the NFC reader 182 receives the request for the NFC tag (T) information and the position information of the NFC reader 182, from the workstation 170, the NFC reader 182 may send the NFC tag (T) information and the position information of the NFC reader 182 to the workstation 170.

When the workstation 170 receives the NFC tag (T) information and the position information of the NFC reader 182, from the NFC reader 182, the workstation 170 may acquire the ID information of the X-ray detector 130 and the storage position information of the corresponding X-ray detector 130, and determine which X-ray detector 130 is stored in which detector storage portion 150 based on the information.

Meanwhile, according to embodiments, the workstation 170 may additionally request the charging information of the X-ray detector 130, to the X-ray detector 130. The charging information of the X-ray detector 130 may include remaining amount information of the battery 135 of the X-ray detector 130.

When the X-ray detector 130 receives a request for the charging information of the X-ray detector 130, from the workstation 170, the X-ray detector 130 may send the charging information of the X-ray detector 130 to the workstation 170. The charging information of the X-ray detector 130 may include the ID information of the X-ray detector 130.

When the workstation 170 receives the charging information of the X-ray detector 130, from the X-ray detector 130, the workstation 170 may display the charging information of the X-ray detector 130 on the display 172 of the workstation 170, based on the received information.

FIG. 18 is a view illustrating a signal flow of the X-ray detector 130 and the workstation 170 in accordance with another embodiment. FIG. 18 illustrates a signal flow of the X-ray imaging apparatus 100b of FIGS. 10 and 11.

Referring to FIG. 18, the workstation 170 may request the sensor value information at a predetermined time interval, to the sensing portion 190. The sensing portion 190 may include the first sensing portion 190-1 installed in the first detector storage portion 151 and the second sensing portion 190-2 installed in the second detector storage portion 152. The sensor value information may include "the size information of the X-ray detector 130" and "the position information of the sensing portion 190" collected by the sensing portion 190.

When the X-ray detector 130 is stored in the first and second detector storage portion 151 and 152, the sensor value may vary, and in this case, the sensing portion 190 may send the sensor value information and the position information of the sensing portion 190 at the request from the workstation 170.

When the workstation 170 receives the sensor value information and the position information of the sensing portion 190, from the sensing portion 190, the workstation 170 may send a ping signal to the first and second detector storage portion 151 and 152. The workstation 170 may send a ping signal to the first and second detector storage portion 151 and 152 in a broadcast manner.

When the X-ray detector 130 is stored in the detector storage portion 150, the X-ray detector 130 may receive the ping signal from the workstation 170. When the X-ray detector 130 receives the ping signal, the X-ray detector 130 may send an ack signal indicating that the ping signal is received, to the workstation 170.

When the workstation 170 receives the ack signal from the X-ray detector 130, the workstation 170 may request the firmware information of the X-ray detector 130, to the X-ray detector 130. The firmware information of the X-ray detector 130 may include "the size information of the X-ray detector 130" and "the ID information of the X-ray detector 130".

When the X-ray detector 130 receives the request for the firmware information of the X-ray detector 130, from the workstation 170, the X-ray detector 130 may send the firmware information of the X-ray detector 130, to the workstation 170.

When the workstation 170 receives the firmware information of the X-ray detector 130, from the X-ray detector 130, the workstation 170 may match "the size information of the X-ray detector 130" acquired from the sensing portion 190, with "the size information of the X-ray detector 130" contained in the firmware information of the X-ray detector 130, and then the workstation 170 may determine which X-ray detector 130 is stored in which detector storage portion 150

Meanwhile, according to embodiments, the workstation 170 may additionally request the charging information of the X-ray detector 130, to the X-ray detector 130. The charging information of the X-ray detector 130 may include remaining amount information of the battery 135 of the X-ray detector 130.

When the X-ray detector 130 receives a request for the charging information of the X-ray detector 130, from the workstation 170, the X-ray detector 130 may send the charging information of the X-ray detector 130 to the workstation 170. The charging information of the X-ray detector 130 may include the ID information of the X-ray detector 130.

When the workstation 170 receives the charging information of the X-ray detector 130, from the X-ray detector 130, the workstation 170 may display the charging information of the X-ray detector 130 on the display 172 of the workstation 170, based on the received information.

As mentioned above, the workstation 170 may sequentially request the firmware information of the X-ray detector 130 and the charging information of the X-ray detector 130, to the X-ray detector 130, but alternatively, the workstation 170 may simultaneously request the firmware information of the X-ray detector 130 and the charging information of the X-ray detector 130, to the X-ray detector 130.

While the present disclosure has been particularly described with reference to exemplary embodiments, it should be understood by those of skilled in the art that various changes in form and details may be made without departing from the spirit and scope of the present disclosure.

## Claims

1. An X-ray detector stored in any one of a plurality of detector storage portions provided in an X-ray imaging apparatus, the X-ray detector comprising:
a detecting portion configured to collect storage position information of the X-ray detector; and
a communicator configured to transmit the storage position information to the X-ray imaging apparatus.

2. The X-ray detector of claim 1, wherein
the detecting portion comprises at least one of an acceleration sensor configured to collect information about a gradient of the X-ray detector and a magnetic sensor configured to collect information about magnetic flux around the X-ray detector.

3. The X-ray detector of claim 1, wherein
the detector storage portion comprises a first detector storage portion and a second detector storage portion adjacent to the first detector storage portion.

4. The X-ray detector of claim 3, wherein
the first detector storage portion is provided to be inclined at a predetermined first angle with the second detector storage portion.

5. The X-ray detector of claim 3, wherein
a magnet is provided between the first detector storage portion and the second detector storage portion.

6. The X-ray detector of claim 1, further comprising:
a memory configured to store identification (ID) information of the X-ray detector.

7. An X-ray imaging apparatus comprising:
a plurality of detector storage portions configured to store an X-ray detector; and
a controller when the X-ray detector is stored in the detector storage portion, configured to determine the corresponding X-ray detector is stored in which detector storage portion between the plurality of detector storage portions, based on position information of the X-ray detector, which is transmitted from the X-ray detector.

8. The X-ray imaging apparatus of claim 7, wherein
the X-ray detector comprises a detecting portion configured to collect position information of the X-ray detector and a communicator configured to transmit the position information to the X-ray imaging apparatus,
wherein the detecting portion comprises at least one of an acceleration sensor configured to collect information about a gradient of the X-ray detector and a magnetic sensor configured to collect information about magnetic flux around the X-ray detector.

9. The X-ray imaging apparatus of claim 8, wherein
the controller determines a storage position of the X-ray detector based on at least one of the gradient information and the magnetic flux information collected by the detecting portion.

10. The X-ray imaging apparatus of claim 7, wherein
the plurality of detector storage portions comprises a first detector storage portion and a second detector storage portion adjacent to the first detector storage portion.

11. The X-ray imaging apparatus of claim 10, wherein
the first detector storage portion is provided to be inclined at a predetermined first angle with the second detector storage portion.

12. The X-ray imaging apparatus of claim 7, wherein
a magnet is provided between the plurality of detector storage portions.

13. The X-ray imaging apparatus of claim 7, wherein
the controller matches ID information of the X-ray detector, which is pre-stored, with ID information of the X-ray detector, which is transmitted from the X-ray detector, and determines a storage position of the X-ray detector.

14. The X-ray imaging apparatus of claim 7, further comprising:
a display configured to display charging information of the X-ray detector stored in the detector storage portion.

15. A control method of an X-ray imaging apparatus having a plurality of detector storage portions in which an X-ray detector is stored, the control method comprising:
receiving ID information and storage position information of the X-ray detector, from the X-ray detector;
determining the X-ray detector is stored in which detector storage portion between the plurality of detector storage portions, based on the ID information and the storage position information of the X-ray detector; and
displaying charging information of the X-ray detector stored in the detector storage portion.
